# EUROPÄISCHE PATENTANMELDUNG

(11) **EP 1 915 942 A1**
(43) Veröffentlichungstag der Anmeldung: **30.04.2008**
(21) Anmeldenummer: 06405453.9
(22) Anmeldetag: 24.10.2006
(51) Int. Cl.: A61B 3/14, A61B 3/13, A61B 3/135

(54) **Optische Erfassung eines Gegenstands**

(71) Anmelder: HAAG-STREIT AG, 3098 Köniz (CH)
(72) Erfinder: Schurter, Walter, 3098 Köniz (CH)
(74) Vertreter: Rüfenacht, Philipp Michael

(57) **Zusammenfassung**

Bei dem erfindungsgemässen Verfahren bzw. der entsprechenden Vorrichtung (1) zur optischen Erfassung eines Gegenstandes (2) wird von dem Gegenstand (2) mit einer Bilderfassungsvorrichtung (15) eine Bewegtbildsequenz aufgenommen, die Bewegtbildsequenz wird von der Bilderfassungsvorrichtung (15) als kontinuierlicher Datenstrom zu einer Datenverarbeitungsvorrichtung (20) übertragen, von der Datenverarbeitungsvorrichtung (20) wird der Datenstrom in Echtzeit in eine Serie von Einzelbildern umgewandelt und die Einzelbilder werden in einem digitalen Bildformat bereitgestellt. Hierbei werden die Einzelbilder in Echtzeit in einem Ringspeicher (26) der Datenverarbeitungsvorrichtung (20) mit n Speicherplätzen (27.1, 27.2, ..., 27.24) abgespeichert, wobei das neuste Einzelbild in einem nächsten, freien Speicherplatz des Ringspeichers (26) abgespeichert wird, solange in dem Ringspeicher (26) freie Speicherplätze vorhanden sind, und, sobald alle n Speicherplätze des Ringspeichers (26) belegt sind, das jeweils älteste Einzelbild mit dem jeweils neusten Einzelbild überschrieben wird.

## Beschreibung

### Technisches Gebiet

Die Erfindung betrifft ein Verfahren zur optischen Erfassung eines Gegenstandes, wobei von dem Gegenstand mit einer Bilderfassungsvorrichtung eine Bewegtbildsequenz aufgenommen wird, die Bewegtbildsequenz von der Bilderfassungsvorrichtung als kontinuierlicher Datenstrom zu einer Datenverarbeitungsvorrichtung übertragen wird, von der Datenverarbeitungsvorrichtung der Datenstrom in Echtzeit in eine Serie von Einzelbildern umgewandelt wird und die Einzelbilder in einem digitalen Bildformat bereitgestellt werden. Weiter betrifft die Erfindung eine entsprechende Vorrichtung.

### Stand der Technik

In vielen Bereichen des täglichen Lebens werden heute Bilder von Gegenständen, Objekten, Personen, Landschaften etc. gemacht. Der Zweck solcher Bilder kann sehr unterschiedlich sein. Bei Urlaubsfotos geht es typischerweise darum, eine bestimmte Stimmung, eine schöne Landschaft oder einfach eine bestimmte Situationen einzufangen, um sie sich später selber noch einmal anzusehen oder sie anderen Personen präsentieren zu können. In anderen Bereichen wie z. B. in der Industrie, können Bilder dazu verwendet werden, Prozesse zu überwachen und zu steuern oder bestimmte Qualitätsüberprüfungen durchzuführen. Durch einzelne Bilder können auch Momentaufnahmen von Prozessen oder Bewegungsabläufen gemacht werden, welche es erlauben, den Prozess anhand von stehenden Aufnahmen in Ruhe zu untersuchen. Auch in der Medizin werden häufig Bilder angefertigt. Mit Bildern kann beispielsweise der Zustand eines Patienten oder, etwa wenn zeitlich versetzt mehrere Aufnahmen eines Patienten gemacht werden, der Verlauf einer Krankheit dokumentiert werden. Die Bilder können beispielsweise auch zur Begutachtung und/oder zur Mithilfe bei der Behandlung eines Patienten an andere Fachpersonen weitergeleitet werden, welche sich nicht in der Nähe des Patienten, sondern weit von diesem entfernt befinden (Telemedizin), wobei die Übermittlung sowohl elektronisch als auch in Form von Abzügen und sowohl online als auch offline erfolgen kann. Ein Beispiel aus der Medizin betrifft die Augenheilkunde, in der Fachsprache Ophthalmologie genannt. Bei Augenuntersuchungen werden sehr häufig Aufnahmen eines Auges eines Patienten angefertigt, sei es zu Dokumentations- oder Diagnosezwecken, zur Übermittlung an weitere Personen oder sonst irgendwelchen Gründen.

Zur Aufnahme von Bildern gibt es viele verbreitete Möglichkeiten wie analoge oder digitale Fotokameras der unterschiedlichsten Ausbildungen. Einzelne Bilder können aber auch mit Film- oder Videokameras gemacht werden, indem diese beispielsweise in einem entsprechenden Foto-Modus betrieben werden oder indem aus der aufgenommenen Film- oder Videosequenz eine einzelne Aufnahme extrahiert wird. Dieser Vorgang wird als "frame grabbing" bezeichnet.

Im Bereich der Medizin ist aus der WO 00/71020 A1 beispielsweise ein Ophthalmoskop bekannt, welches einen Bildsensor umfasst, der ein Videosignal erzeugt. Dieses wird via ein Kabel an einen Computer übermittelt und dort auf einem Bildschirm dargestellt. Durch Drücken eines Knopfes kann der Operator die Aufnahme eines Einzelbildes auslösen. Mit Hilfe einer Video Grabber Karte wird dann aus dem Videosignal das gewünschte Bild abgegriffen, in einem Speicher abgelegt und auf dem Bildschirm als Standbild dargestellt.

Bei der Aufnahme von stehenden Bildern (Fotografien) passiert es allerdings immer wieder, dass das Bild im falschen Moment aufgenommen wird, d. h. dass die Person, welche die Aufnahme macht, den Auslöser im falschen Moment betätigt oder dass die Kamera, mit welcher die Aufnahme gemacht wird, eine (unberechenbare) Auslöseverzögerung aufweist, so dass die Aufnahme zu spät oder allenfalls auch zu früh gemacht wird. Fast jeder kennt z. B. das Problem, dass genau in dem Moment, in welchem ein bestimmtes Motiv fotografiert wird, eine Drittperson vor der Kamera vorbeiläuft und statt des Motivs die Drittperson aufgenommen wird. Oder allgemeiner ausgedrückt, dass sich das aufzunehmende Motiv oder dessen Sichtbarkeit für kurze Zeit ändert und die Aufnahme genau in einem solchen kurzen Moment gemacht wird, in welchem sich das Motiv nicht in dem gewünschten Zustand befindet oder in welchem es nicht in der gewünschten Art und Weise sichtbar ist.

In der Ophthalmologie passiert es beispielsweise immer wieder, dass sich der Patient genau in dem Moment bewegt, in welchem eine Aufnahme seines Auges gemacht wird. Wenn der Patient beispielsweise genau im falschen Moment blinzelt, ist auf der entsprechenden Aufnahme nicht sein Auge, sondern sein Augenlid zu sehen. Es kann auch sein, dass irgendwelche Lichtreflexe oder beliebige andere Ereignisse die Aufnahme stören und diese so unbrauchbar machen. Dieses Problem besteht auch bei der oben erwähnten WO 00/71020 A1. Zwischen dem Drücken des Knopfes durch den Operator und dem Moment, in welchem die Aufnahme gemacht wird, vergeht typischerweise eine bestimmte Zeit. Wenn der Patient genau dann blinzelt, kann diese Aufnahme nicht verwendet und muss wiederholt werden. Hierbei nützt es auch nichts, wenn die Auslöseverzögerung verkürzt wird, da man nicht voraussehen kann, wann der Patient das nächste Mal blinzelt.

Ein ähnliches Problem ergibt sich aber auch im umgekehrten Fall, wenn etwa der aufzunehmende Moment nur sehr kurze Zeit andauert. Wenn der gewünschte Moment beispielsweise nur Sekundenbruchteile dauert, ist es sehr schwierig, wenn nicht gar unmöglich, die Kamera so zu bedienen, dass die Aufnahme genau in dem gewünschten Moment gemacht wird.

### Darstellung der Erfindung

Aufgabe der Erfindung ist es daher, ein dem eingangs genannten technischen Gebiet zugehörendes Verfahren bzw. eine entsprechende Vorrichtung zu schaffen, welches die erwähnten Nachteile vermeidet und es erlaubt, eine Aufnahme eines Gegenstandes in einem bestimmten Moment zu machen, sodass die Aufnahme den Gegenstand in dem gewünschten Zustand zeigt.

Die Lösung der Aufgabe ist durch die Merkmale des Anspruchs 1 definiert. Gemäss der Erfindung umfasst das Verfahren zur optischen Erfassung eines Gegenstandes die folgenden Schritte:
a) von dem Gegenstand wird mit einer Bilderfassungsvorrichtung eine Bewegtbildsequenz aufgenommen,
b) die Bewegtbildsequenz wird von der Bilderfassungsvorrichtung als kontinuierlicher, insbesondere digitaler, Datenstrom zu einer Datenverarbeitungsvorrichtung übertragen,
c) von der Datenverarbeitungsvorrichtung wird der Datenstrom in Echtzeit in eine Serie von Einzeibildern umgewandelt und
d) die Einzelbilder werden in einem digitalen Bildformat bereitgestellt,
e) die Einzelbilder werden dann in Echtzeit in einem Ringspeicher der Datenverarbeitungsvorrichtung mit n Speicherplätzen abgespeichert,
f) wobei das neuste Einzelbild in einem nächsten, freien Speicherplatz des Ringspeichers abgespeichert wird, solange in dem Ringspeicher freie Speicherplätze vorhanden sind,
g) und, sobald alle n Speicherplätze des Ringspeichers belegt sind, das jeweils älteste Einzelbild mit dem jeweils neusten Einzelbild überschrieben wird.

Auf diese Weise sind in dem Ringspeicher jeweils immer die letzten n Bilder des Gegenstandes abgespeichert, wobei jedes einzelne Bild für die weitere Verarbeitung aus dem Ringspeicher ausgelesen werden kann. n ist hierbei eine ganze Zahl grösser als Null. Wird der Ringspeicher genügend gross gewählt, beispielsweise so, dass er zur Speicherung von 30 Bildern ausreicht, und beträgt die Bildrate des Bilderfassungsgeräts beispielsweise zehn Bilder pro Sekunde, befinden sich in dem Ringspeicher jeweils immer sämtliche Aufnahmen der letzten drei Sekunden. Dies gilt allerdings nicht für die allerersten drei Sekunden, in welchen der zunächst völlig leere Ringspeicher zum ersten Mal gefüllt wird.

Durch die Erfindung kann viel Zeit eingespart werden, denn meistens ist es nicht mehr notwendig, eine bestimmte Aufnahme zu wiederholen, wenn sie aus einem der erwähnten Gründe unbrauchbar ist. Wird von einem Gegenstand beispielsweise eine Film- oder Videosequenz von einer Sekunde Länge aufgenommen, und sind von diesen zehn Bildern eines oder auch mehr unbrauchbar, kann einfach eines der anderen Bilder für die weitere Verarbeitung verwendet werden. Wenn man umgekehrt ein Ereignis in einem Bild festhalten will, welches z. B. nur ein oder zwei Zehntels Sekunden dauert, startet man die Kamera bevor das Ereignis stattfindet, und filmt solange, bis das Ereignis stattgefunden hat, worauf die Kamera gestoppt wird und sich zumindest ein Bild des Ereignisses im Ringspeicher befindet. Ist die Ereignisdauer kürzer als eine Zehntels Sekunde, kann einfach eine Kamera mit einer höheren Bildrate verwendet werden, so dass pro Sekunde mehr als zehn Bilder aufgenommen werden können.

Je nach Anwendung kann auch der Aufwand und damit die Kosten zur Erstellung des Bildes gesenkt werden, beispielsweise wenn der abzubildende Gegenstand vor der Aufnahme in irgend einer Weise präpariert werden muss, damit die gewünschte Aufnahme auch tatsächlich gemacht werden kann.

Als Bilderfassungsvorrichtung ist im Prinzip jedes Gerät geeignet, das eine Film- oder Videosequenz aufnehmen und in analoger oder digitaler Form ausgeben kann. Für die weitere Verarbeitung ist es dann von Vorteil, wenn die Bildsequenz in Form digitaler Daten vorliegt, da sie auf diese Weise von der Datenverarbeitungsvorrichtung, beispielsweise einem Computer, in optimaler Weise verarbeitet werden kann. Selbstverständlich ist es aber auch möglich, dass die Kamera die Daten in analoger Form erstellt und die Daten dann in der Kamera, im Computer oder mit einer zusätzlichen Vorrichtung digitalisiert werden. Die Kamera und der Computer sind typischerweise zwei verschiedene Geräte, die über entsprechende Datenübertragungsvorrichtungen wie Kabel oder Funkverbindungen miteinander verbunden werden. Allerdings können sie auch zu einem einzigen Gerät vereint sein.

Die Erfindung kann im Prinzip überall eingesetzt werden, wo von einem Gegenstand, einem Objekt, einer Person, einem Vorgang etc. ein einzelnes Bild gemacht werden muss, wo allerdings die Rahmenbedingungen nicht zu hundert Prozent kontrolliert und beeinflusst werden können, so dass eine gewisse Wahrscheinlichkeit besteht, dass eine erstellte Aufnahme für den beabsichtigen Zweck unbrauchbar ist.

Ein bevorzugtes Ausführungsbeispiel der Erfindung betrifft die Ophthalmologie, und zwar die optische Erfassung des Auges eines Patienten. Wie bereits erwähnt, sind solche Aufnahmen manchmal nicht zu gebrauchen, weil der Patient z. B. genau im Moment der Aufnahme geblinzelt hat.

Das Blinzeln dauert typischerweise rund eine Zehntels Sekunde oder etwas länger. D. h. wenn das Auge des Patienten eine Sekunde lang gefilmt wird und der Patient genau innerhalb dieser Sekunde blinzelt, ist auf einem oder zwei der 10 Bilder das Blinzeln zu sehen, aber nicht auf den restlichen acht oder neun Bildern. Selbst wenn der Patient also während der Aufnahme, welche lediglich eine Sekunde lang dauert, blinzelt, muss die Aufnahme nicht wiederholt werden, da sich im Ringspeicher ja acht oder neun Aufnahmen befinden, auf welchen der Patient nicht blinzelt. D. h. es kann viel Zeit eingespart werden, da die Kamera nicht ein zweites oder noch mehr Male vor dem Auge des Patienten justiert werden muss. Damit kann aber auch die Belästigung des Patienten verringert werden, insbesondere wenn der Patient zur Erstellung der Aufnahme in einer bestimmten Art und Weise vorbehandelt werden muss, beispielsweise wenn das Auge durch bestimmte Medikamente unempfindlich gemacht werden muss oder wenn optische Vorrichtungen wie etwa ein Kontaktglas auf dem Auge platziert werden müssen. Auch die Zeitdauer, während welcher sich der Patient nicht bewegen darf, kann verkleinert werden.

Der Operator muss typischerweise den optimalen Zeitpunkt für das Ein- und/oder Ausschalten der Kamera bestimmen und/oder er muss die Kamera optimal vor dem abzubildenden Gegenstand positionieren. Je nach Anwendung und/oder Erfahrung kann dies ein Operator machen, ohne die Bilder zu sehen, die im Ringspeicher abgespeichert werden. Bei bestimmten Anwendungen ist es jedoch von Vorteil, wenn der Operator die Bilder, die im Ringspeicher abgelegt werden, auch sieht. Bei einer bevorzugten Ausführungsform der Erfindung werden die Einzelbilder daher in Echtzeit auf einer Bildanzeigevorrichtung der Datenverarbeitungsvorrichtung angezeigt. Dies wird typischerweise ein an einen Computer angeschlossener Bildschirm sein, wobei es auch möglich ist, die Bilder in Echtzeit auf einem in die Kamera integrierten oder an diese angeschlossenen Monitor anzuzeigen.

Ist das aufzunehmende Ereignis vorbei und auf einem oder mehreren der im Ringspeicher abgelegten Bilder drauf, darf dieses Bild bzw. dürfen diese Bilder nicht mehr überschrieben werden. Es ist daher von Vorteil, wenn das Aufnehmen der Bewegtbildsequenz gestoppt wird, sobald das gewünschte Ereignis vorüber ist und im Ringspeicher die neusten n Einzelbilder des Gegenstandes abgespeichert sind. Zwar kann die Aufnahme auch automatisch gestoppt werden, aber das Stoppen der Aufnahme erfolgt mit Vorteil über eine Auslösevorrichtung wie z. B. einen Knopf oder einen Hebel, welcher vom Operator betätigt werden muss. Wie schnell dies geschehen muss, hängt unter anderem von der Länge des Ringspeichers ab. Je grösser also die Zahl n der im Ringspeicher speicherbaren Bilder, desto länger hat der Operator Zeit, die Aufnahme zu stoppen, ohne dass das gewünschte Bild oder die gewünschten Bilder mit neueren Bildern überschrieben werden.

Bei einem besonders bevorzugten Ausführungsbeispiel der Erfindung können die im Ringspeicher abgespeicherten Einzelbilder einzeln und in beliebiger Reihenfolge auf dem Bildschirm angezeigt werden. Der Operator kann die Bilder beispielsweise in der aufgenommenen oder auch in der umgekehrten Reihenfolge durchblättern. Er kann aber auch gezielt ein beliebiges Bild anzeigen lassen. Diese Steuerung erfolgt beispielsweise über die Tastatur oder andere am Computer angeschlossene Eingabegeräte wie etwa eine Maus, ein Touchpad oder dergleichen.

Indem der Operator durch die im Ringspeicher abgespeicherten Bilder durchblättert, kann er diese Bild für Bild begutachten. Vorzugsweise kann dann der Operator dasjenige Bild bestimmen und für die weitere Bearbeitung auswählen, das die von ihm für die gewünschte Aufnahme aufgestellten Kriterien am besten erfüllt. Die weitere Bearbeitung kann dabei nicht nur eine dauerhafte Speicherung umfassen, sondern beispielsweise auch die Übermittlung des ausgewählten Bildes in elektronischer Form an eine weitere Datenverarbeitungsvorrichtung, aber auch das Ausdrucken auf einer entsprechenden Druckvorrichtung.

Die Bildrate, mit welcher die Bilderfassungsvorrichtung die Bewegtbildsequenz des Gegenstandes aufnimmt, ist im Prinzip beliebig wählbar, hängt allerdings von der jeweiligen Anwendung sowie von den jeweiligen technischen Voraussetzungen bzw. Möglichkeiten der Bilderfassungsvorrichtung, der Datenverarbeitungsvorrichtung und der diese beiden verbindenden Kommunikationsmittel ab. Die Bildrate ist jedoch typischerweise grösser als 5 Bilder pro Sekunde und liegt mit Vorteil zwischen 10 und 30 Bildern pro Sekunde, vorzugsweise zwischen 10 und 20 Bildern pro Sekunde. Bildraten von bis zu 30 Bildern pro Sekunde sind mit handelsüblichen Videokameras und Computern ohne weiteres erreichbar.

In der Ophthalmologie gibt es eine Vielzahl von Instrumenten und Untersuchungsgeräten zur Untersuchung eines Auges. Es gibt die verschiedensten Geräte zur Untersuchung der vorderen Augenabschnitte oder des Augenhintergrundes wie auch der seitlichen Bereiche. Als Beispiel sei hier ein Spaltlampenmikroskop erwähnt, welches für verschiedene, optische Untersuchungen eingesetzt werden kann. Viele dieser Untersuchungsgeräte sind mit entsprechenden Vorrichtungen zur optimalen Positionierung des Gerätes vor dem Auge ausgerüstet. Sie umfassen beispielsweise einen so genannten Kreuzschlitten, auf welchem das Untersuchungsgerät befestigt ist und welcher eine geführte und präzise Bewegung des Untersuchungsgerätes in allen drei Raumrichtungen ermöglicht. Bei der Anwendung der Erfindung in der Ophthalmologie wird die Bilderfassungsvorrichtung daher vorzugsweise auf dem Untersuchungsgerät montiert. Dies geschieht mit Vorteil so, dass eine optische Achse der Bilderfassungsvorrichtung mit einer optischen Achse des Untersuchungsgeräts zusammenfällt.

Umfasst das Untersuchungsgerät auch eine Positioniervorrichtung wie eben erwähnt, wird die Bilderfassungsvorrichtung in vorteilhafter Weise natürlich auch mittels dieser Positioniervorrichtung vor dem zu untersuchenden Auge des Patienten positioniert.

Die im Ringspeicher abgespeicherten Bilder können, wie vorgängig erwähnt, einzeln nacheinander betrachtet werden, wobei das optimale Bild für die weitere Bearbeitung ausgewählt werden kann. Bei anderen Anwendungen ist das gewünschte Ergebnis jedoch nicht ein einzelnes Bild, sondern eine Bildsequenz. Bei einer weiteren bevorzugten Ausführungsvariante der Erfindung werden daher wenigstens zwei, typischerweise jedoch ein Grossteil oder gar sämtliche der im Ringspeicher abgespeicherten Einzelbilder zu einer Bewegtbildsequenz zusammengefügt, welche ihrerseits wieder abgespeichert oder an jemanden weitergeleitet werden kann.

Eine Vorrichtung zur optischen Erfassung eines Gegenstandes umfasst eine Bilderfassungsvorrichtung und eine Datenverarbeitungsvorrichtung mit einem Speicher. Mit der Bilderfassungsvorrichtung ist von dem Gegenstand eine Bewegtbildsequenz aufnehmbar und in Form eines kontinuierlichen, insbesondere digitalen, Datenstroms zur Datenverarbeitungsvorrichtung übertragbar. Hierbei ist der Datenstrom von der Datenverarbeitungsvorrichtung in Echtzeit in eine Serie von Einzelbildern umwandelbar und die Einzelbilder sind in einem digitalen Bildformat bereitstellbar. Erfindungsgemäss ist eine solche Vorrichtung nun derart ausgebildet, dass die Einzelbilder von der Datenverarbeitungsvorrichtung in Echtzeit in dem Speicher abspeicherbar sind. Der Speicher ist hierbei als Ringspeicher mit n Speicherplätzen ausgebildet, so dass das jeweils neuste Einzelbild in einem nächsten, freien Speicherplatz des Ringspeichers abspeicherbar ist, solange in dem Ringspeicher freie Speicherplätze vorhanden sind, und dass jeweils das älteste Einzelbild mit dem jeweils neusten Einzelbild überschreibbar ist, sobald alle n Speicherplätze des Ringspeichers belegt sind.

Wie bereits erwähnt, kann die Erfindung prinzipiell mit beliebigen Bilderfassungsvorrichtungen implementiert werden. Da eine Videokamera allerdings bereits ein elektronisches Ausgangssignal liefert, wird der Einsatz einer Videokamera bevorzugt. Es werden insbesondere Videokameras bevorzugt, welche einen digitalen Datenstrom der Bewegtbildsequenz ausgeben, da hierbei die A/D Umwandlung nicht separat ausgeführt werden muss.

Ein weiterer Vorteil kann erzielt werden, wenn die Bilderfassungsvorrichtung elektronisch steuerbar ist und nicht wie die meisten Kameras für den Hausgebrauch manuell bedient werden muss. Die Steuerung kann dadurch viel präziser und auch reproduzierbarer durchgeführt werden. Dabei ist die Bilderfassungsvorrichtung in einer bevorzugten Ausführungsform der Erfindung von der Datenverarbeitungsvorrichtung aus steuerbar. D. h. auf einem Computer ist beispielsweise eine Software installiert, mit welcher sich entsprechende Steuersignale erzeugen und an die Kamera übermitteln lassen. Aber nicht nur der Computer muss entsprechend ausgerüstet sein, auch die Kamera muss hierfür geeignet und über eine entsprechende Kommunikationsverbindung an den Computer angeschlossen sein.

Die Anzahl n der Speicherplätze im Ringspeicher kann fest vorgegeben sein. Mit Vorteil ist sie allerdings konfigurierbar, so dass je nach Anwendung eine passende Anzahl Einzelbilder im Ringspeicher abgelegt werden können. Die Anzahl n der Speicherplätze hängt dabei z. B. von der Gesamtgrösse des zur Verfügung stehenden Speichers ab. Aber auch die gewünschte Auflösung, d. h. die Anzahl Bildpunkte, oder auch die Farbtiefe des Bildes, können einen Einfluss auf die Anzahl speicherbarer Einzelbilder haben. Je grösser die Auflösung und je höher die Farbtiefe, desto weniger Bilder können in einem Speicher bestimmter Grösse gespeichert werden.

Als Ringspeicher kann an sich jede beliebige Speichertechnologie (elektronisch, magnetisch, optisch, magneto-optisch etc.) eingesetzt werden, welche für die elektronische Speicherung von Bilddaten geeignet ist. Es können auch die unterschiedlichsten Speichertypen wie z. B. Festplattenspeicher, Flash-Speicher, Disketten oder auch beschreibbare Compact Discs (CD) oder Digital Versatile Discs (DVD) verwendet werden; es muss lediglich möglich sein, die erforderliche Anzahl Bilder pro Sekunde, welche von der Kamera geliefert werden, in Echtzeit zu speichern. Aus diesem Grund werden schnelle Speichertechnologien wie z. B. die flüchtigen RAM (Random Access Memory) Speicher von Computern bevorzugt als Ringspeicher eingesetzt.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: Eine erfindungsgemässe Vorrichtung zur optischen Erfassung eines Auges eines Patienten;
- Fig. 2: eine schematische Darstellung des Ringspeichers als Teil des RAM-Speichers des Computers aus Fig. 1 und
- Fig. 3: eine weitere Darstellung des Ringspeichers zur Verdeutlichung dessen Funktionsweise.

Grundsätzlich sind in den Figuren gleiche Teile mit gleichen Bezugszeichen versehen.

### Wege zur Ausführung der Erfindung

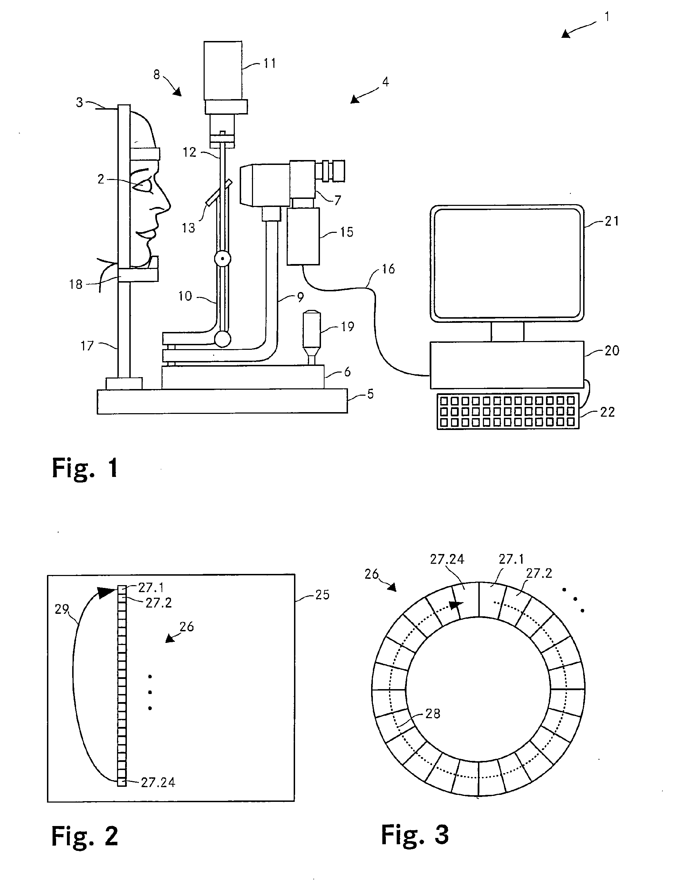

Fig. 1 zeigt eine erfindungsgemässe Vorrichtung 1 zur optischen Erfassung eines Auges 2 eines Patienten 3. Die Vorrichtung umfasst ein Spaltlampenmikroskop 4 mit einem Tisch 5, einem Kreuzschlitten 6, einem Mikroskop 7 und einer Beleuchtungseinheit 8. Das Mikroskop 7 sitzt auf einem L-förmigen Haltearm 9, der drehbar mit dem Kreuzschlitten 6 verbunden ist. Entsprechend ist auch die Beleuchtungseinheit 8 über einen L-förmigen Haltearm 10 drehbar auf dem Kreuzschlitten 6 befestigt. Die Beleuchtungseinheit 8 wiederum umfasst ein Leuchtvorrichtung 11, welche über ein Gestänge 12 drehbar mit dem Haltearm 10 verbunden ist, so dass das von der Leuchtvorrichtung 11 ausgehende Licht von dem Spiegel 13 unter verschiedenen Winkeln auf das Auge 2 gerichtet werden kann.

Die Vorrichtung 1 umfasst zudem eine Videokamera 1 5, sowie einen Computer 20 mit einem Bildschirm 21 und einer Tastatur 22. Die Videokamera 15 ist über einen entsprechenden Adapter in bekannter Weise an das Mikroskop 7 angeschlossen, so dass beispielsweise einer der beiden Strahlengänge des binokularen Mikroskops 7 anstatt auf eines der Okulare des Mikroskops 7 bzw. zusätzlich dazu ausgekoppelt und zur Videokamera 15 geführt wird. Die Videokamera 15 ist hierbei derart auf dem Mikroskop 7 befestigt, dass die optische Achse der Videokamera 15 nach der Umlenkung innerhalb des Mikroskops 7 in Richtung des Auges 2 mit der optischen Achse des entsprechenden Strahlengangs des Mikroskops 7 zusammenfällt. Weiter ist die Videokamera über eine Kommunikationsverbindung, in diesem Fall ein Kabel 16, mit dem Computer 20 verbunden.

Auf dem Tisch 5 ist weiter eine Halterung 17 mit einer Kinnstütze 18 befestigt.

Zunächst wird der Kopf des Patienten 3 so in der Halterung 9 positioniert, dass das Kinn des Patienten auf der Kinnstütze 10 abgestützt ist. Nun wird das Mikroskop 7 mit Hilfe des Kreuzschlittens 6 so vor dem Auge 2 positioniert, dass der aufzunehmende Augenabschnitt mit dem Mikroskop 7 in gewünschter Weise abgebildet wird. Hierbei kann es sich um einen beliebigen Augenabschnitt handeln, also sowohl um einen vorderen Augenabschnitt, als auch um den Augenhintergrund oder einen seitlichen Bereich des Auges 2, wobei unter Umständen entsprechende Hilfsmittel, beispielsweise zusätzliche Linsen oder andere optische Geräte oder Instrumente, an einem geeigneten Ort in den Strahlengang eingefügt werden müssen. Die Positionierung des Kreuzschlittens 6 erfolgt beispielsweise mit Hilfe des Hebels 19, mit welchem sich der Kreuzschlitten 6 in allen drei Raumrichtungen bewegen lässt. Die optimale Positionierung lässt sich durch das Okular bzw. die Okulare des Mikroskops 7 kontrollieren. Oder, was bequemer ist, die Videokamera 15 wird bereits vor dem Positionieren eingeschaltet und die mit der Videokamera 15 aufgenommenen Bilder werden auf dem Bildschirm 21 in Echtzeit dargestellt. Dann kann der Operator das jeweilige Resultat der Abbildung direkt am Bildschirm 21 mitverfolgen und so sehr einfach die gewünschte Position des Mikroskops 7 bestimmen und einstellen.

Bildet das Mikroskop 7 den gewünschten Augenabschnitt korrekt ab, wird die Videokamera 15 eingeschaltet, falls sie nicht bereits vorher eingeschaltet war. Jetzt kann auch noch der Computer 20 in den entsprechenden Aufnahmezustand versetzt werden, indem dieser z. B. hochgefahren und das entsprechende Programm gestartet wird. So wird die von der Videokamera 15 aufgezeichnete Videosequenz über das Kabel 16 zum Computer 20 übertragen und vom Computer 20 in eine Serie von Einzelbildern umgewandelt. Diese Einzelbilder werden dann in einem Speicher des Computers 20 in Echtzeit zwischengespeichert und auf dem Bildschirm 21 auch in Echtzeit angezeigt. Der Speicher ist im vorliegenden Beispiel ein RAM Speicher (auch als Arbeitsspeicher bezeichnet) des Computers 20, der entsprechend kurze Schreib- und Lesegeschwindigkeiten aufweist. Die Umwandlung des kontinuierlichen Datenstroms der Videokamera 15 in eine Serie von Einzelbildern kann sowohl softwaremässig als auch hardwaremässig erfolgen. Im dargestellten Beispiel erfolgt die Umwandlung mit einer entsprechenden Software, wodurch es möglich ist, die Erfindung ohne entsprechende zusätzliche, teure Hardware zu implementieren. D. h. die Erfindung kann mit einem handelsüblichen Personalcomputer (PC) realisiert werden.

Mit einer auf dem Computer 20 ausgeführten Software kann beispielsweise auch die Videokamera 15 vollständig gesteuert werden. D. h. nebst dem Ein- und Ausschalten der Videokamera 15 kann auch konfiguriert werden, mit welcher Auflösung und mit welchen und wie vielen Farben die Videokamera 15 die Aufnahmen machen soll. Ebenso kann mit einer solchen Software die Anzahl n der zu speichernden Einzelbilder konfiguriert werden.

Fig. 2 zeigt eine schematische Darstellung des RAM-Speichers 25 des Computers 20. Ein Teil des RAM-Speichers 25 ist hierbei quasi reserviert für die Speicherung der Einzelbilder und bildet damit den Ringspeicher 26. Der hierfür notwendige Speicherbedarf wird berechnet aufgrund der Anzahl n der zu speichernden Einzelbilder, der gewünschten Auflösung sowie der gewünschten Farbtiefe der Aufnahmen. Sämtliche dieser Parameter und viele andere zusätzliche Einstellungen sind wie bereits erwähnt konfigurierbar.

In dem dargestellten Beispiel ist die Anzahl n beispielsweise 24 und der Ringspeicher 26 umfasst entsprechend 24 Speicherbereiche 27.1, 27.2, ... 27.24 wobei jeder Speicherbereich 27.1, 27.2, ... 27.24 zur Speicherung eines ganzen Einzelbildes dient.

Da die Einzelbilder in einem bestimmten Daten- bzw. Bildformat in dem Ringspeicher 26 gespeichert werden, sind alle Speicherbereiche 27.1, 27.2, ... 27.24 gleich gross. Der Speicherbedarf für ein Einzelbild hängt allerdings vom Daten- bzw. Bildformat ab, in welchem es gespeichert wird. Grundsätzlich kann praktisch jedes beliebige Bildformat zur Speicherung der Einzelbilder verwendet werden. Dabei sind sowohl Bildformate möglich, bei welchen ein Bild Pixel für Pixel gespeichert wird (Bitmap-Formate, z. B. bmp-Format), aber auch Bildformate, bei welchen diese Rohdaten beispielsweise einer (verlustfreien oder verlustbehafteten) Komprimierung unterworfen werden, sind möglich (z. B. jpg-Format).

Je nach Bildformat, Auflösung und Farbtiefe kann der für die Speicherung eines (für eine ophthalmologische Untersuchung geeigneten) Einzelbildes benötigte Speicherbedarf zwischen einigen tausend Bytes und einigen Megabytes betragen. Werden die Einzelbilder beispielsweise mit einer Auflösung von zwei Megapixeln und einer Farbtiefe von 24 Bit (je acht Bit für jeden von drei Farbkanälen, beispielsweise rot, grün und blau) gespeichert, beträgt die Grösse eines Einzelbildes rund sechs Megabytes (drei mal zwei Megabytes). Bei diesem Beispiel würde folglich jeder Speicherbereich 27.1, 27.2, ... 27.24 eine Grösse von rund sechs Megabytes umfassen.

Das erste Einzelbild, das vom Computer 20 aus dem Datenstrom der Videokamera 15 extrahiert wird, wird beispielsweise im Speicherbereich 27.1 gespeichert. Das zweite Einzelbild im Speicherbereich 27.2 und so weiter, bis das vierundzwanzigste Einzelbild im Speicherbereich 27.24 gespeichert wird. Das fünfundzwanzigste Einzelbild wird dann wieder im Speicherbereich 27.1 gespeichert. D. h. das zuvor in diesem Speicherbereich 27.1 abgespeicherte erste Einzelbild wird mit dem fünfundzwanzigsten Einzelbild überschrieben. Entsprechend wird das sechsundzwanzigste Einzelbild im Speicherbereich 27.2 gespeichert, und so weiter. Diese "ringförmige" Speicherung ist durch den Pfeil 29 angedeutet, der beispielsweise die Art der Adressierung der einzelnen Speicherbereiche 27.1, 27.2, ... 27.24 symbolisiert. Diese Speicherorganisation ist nicht mit einem FIFO (first in first out) Speicher zu verwechseln, bei welchem die Speicherinhalte nach dem Auslesen des jeweils ältesten Speicherinhalts um eine Position weitergeschoben werden.

Fig. 3 zeigt eine etwas anschaulichere Darstellung des Ringspeichers 26, bei welcher die einzelnen Speicherbereiche 27.1, 27.2, ... 27.24 ringförmig angeordnet sind und so die Funktionsweise des Ringspeichers 26 etwas deutlicher wird. Die Einzelbilder werden hierbei der Reihe nach in den Speicherbereichen 27.1, 27.2, ... 27.24 abgespeichert, was wiederum durch den Pfeil 28 angedeutet ist.

Betätigt der Operator nun den Auslöser, der die Aufnahme der Videokamera 15 bzw. die Speicherung der Einzelbilder im Ringspeicher 26 stoppt, befinden sich in diesem Ringspeicher 26 die letzten, d. h. neuesten 24 Einzelbilder. Der Auslöser kann irgend ein Knopf oder Hebel der Vorrichtung 1 sein. Typischerweise wird es eine bestimmte Taste der Tastatur 22 sein. Es wäre allerdings auch möglich, am Untersuchungsgerät (4) einen entsprechenden Knopf vorzusehen, beispielsweise am Hebel 19.

Mit Hilfe einer entsprechenden Software kann nun der Operator jedes einzelne dieser 24 Einzelbilder auswählen und es sich auf dem Bildschirm 21 anzeigen lassen, damit er es ansehen und begutachten kann. Die Reihenfolge ist dabei vom Operator frei wählbar. Er kann sich die Einzelbilder z. B. in der aufgenommenen oder in der umgekehrten Reihenfolge anschauen. Er kann sich aber auch ein beliebiges Einzelbild auf dem Bildschirm 21 anzeigen lassen. Das Ziel dabei ist, dass der Operator dasjenige Einzelbild identifiziert, welches für die weitere Verwendung am besten geeignet ist. Hat er das gewünschte Einzelbild (es können auch mehrere Einzelbilder sein) gefunden, kann er dieses z. B. in einem permanenten Speicher wie etwa auf der Harddisk (nicht dargestellt) des Computers 20 abspeichern. Er kann es aber auch auf einem portablen Speicher ablegen, damit er es mitnehmen kann. Es ist selbstverständlich auch möglich, das Bild über entsprechende Kommunikationsverbindungen zu einem anderen Computer zu senden, damit es sich z. B. auch eine andere Person ansehen kann.

Sämtliche vorgängig erwähnten softwaremässig realisierten Programme können beispielsweise als ein einziges Programmpaket ausgebildet sein, welches auf dem Computer 20 installiert ist und aufgerufen werden kann. Es können aber auch mehrere Softwaremodule sein, die jeweils eine oder mehrere der erwähnten Funktionalitäten umfassen.

Zusammenfassend ist festzustellen, dass die Erfindung mit einem geringen Aufwand erlaubt, eines oder mehrere Einzelbilder eines Gegenstandes zu machen, wobei die Wahrscheinlichkeit, dass ein Bild unbrauchbar ist und die Aufnahme wiederholt werden muss, enorm verkleinert werden kann.

## Patentansprüche

1. Verfahren zur optischen Erfassung eines Gegenstandes (2) mit folgenden Schritten:
a) von dem Gegenstand (2) wird mit einer Bilderfassungsvorrichtung (15) eine Bewegtbildsequenz aufgenommen,
b) die Bewegtbildsequenz wird von der Bilderfassungsvorrichtung (15) als kontinuierlicher, insbesondere digitaler, Datenstrom zu einer Datenverarbeitungsvorrichtung (20) übertragen,
c) von der Datenverarbeitungsvorrichtung (20) wird der Datenstrom in Echtzeit in eine Serie von Einzelbildern umgewandelt und
d) die Einzelbilder werden in einem digitalen Bildformat bereitgestellt,
**dadurch gekennzeichnet, dass**
e) die Einzelbilder in Echtzeit in einem Ringspeicher (26) der Datenverarbeitungsvorrichtung (20) mit n Speicherplätzen abgespeichert werden,
f) wobei das neuste Einzelbild in einem nächsten, freien Speicherplatz (27.1, 27.2, ..., 27.24) des Ringspeichers (26) abgespeichert wird, solange in dem Ringspeicher (26) freie Speicherplätze (27.1, 27.2, ..., 27.24) vorhanden sind,
g) und, sobald alle n Speicherplätze (27.1, 27.2, ..., 27.24) des Ringspeichers (26) belegt sind, das jeweils älteste Einzelbild mit dem jeweils neusten Einzelbild überschrieben wird.

2. Verfahren nach Anspruch 1, wobei ein Auge (2) eines Patienten (3) optisch erfasst wird.

3. Verfahren nach einem der Ansprüche 1-2, wobei die Einzelbilder in Echtzeit auf einer Bildanzeigevorrichtung (21) der Datenverarbeitungsvorrichtung (20) angezeigt werden.

4. Verfahren nach einem der Ansprüche 1-3, wobei das Aufnehmen der Bewegtbildsequenz gestoppt wird, beispielsweise indem eine entsprechende Auslösevorrichtung betätigt wird, so dass im Ringspeicher (26) die neusten n Einzelbilder des Gegenstandes (2) abgespeichert sind.

5. Verfahren nach Anspruch 4, wobei die im Ringspeicher (26) abgespeicherten Einzelbilder einzeln und in beliebiger, von einem Operator vorgebbaren Reihenfolge auf einer Bildanzeigevorrichtung (21) der Datenverarbeitungsvorrichtung (20) angezeigt werden.

6. Verfahren nach Anspruch 5, wobei eines der Einzelbilder aufgrund vorgegebener Kriterien für eine weitere Bearbeitung ausgewählt wird.

7. Verfahren nach einem der Ansprüche 1-6, wobei die Bewegtbildsequenz des Gegenstandes (2) von der Bilderfassungsvorrichtung (15) mit einer Bildrate von über 5 Bildern pro Sekunde, insbesondere zwischen 10 und 30 Bildern pro Sekunde, vorzugsweise zwischen 10 und 20 Bildern pro Sekunde aufgenommen wird.

8. Verfahren nach einem der Ansprüche 1-7, wobei die Bilderfassungsvorrichtung (15) auf einem ophthalmologischen Untersuchungsgerät (4) derart montiert wird, dass eine optische Achse der Bilderfassungsvorrichtung (15) mit einer optischen Achse des Untersuchungsgeräts (4) zusammenfällt, und die Bilderfassungsvorrichtung (15) mittels einer Positioniervorrichtung (6) des Untersuchungsgeräts (4) vor einem Auge (2) eines Patienten (3) positioniert wird.

9. Verfahren nach einem der Ansprüche 1-8, wobei wenigstens zwei der im Ringspeicher (26) abgespeicherten Einzelbilder zu einer Bewegtbildsequenz zusammengefügt werden.

10. Vorrichtung zur optischen Erfassung eines Gegenstandes (2), wobei die Vorrichtung eine Bilderfassungsvorrichtung (15) und eine Datenverarbeitungsvorrichtung (20) mit einem Speicher (25) umfasst und mit der Bilderfassungsvorrichtung (15) von dem Gegenstand (2) eine Bewegtbildsequenz aufnehmbar und in Form eines kontinuierlichen, insbesondere digitalen, Datenstroms zur Datenverarbeitungsvorrichtung (20) übertragbar ist, wobei der Datenstrom von der Datenverarbeitungsvorrichtung (20) in Echtzeit in eine Serie von Einzeibildern umwandelbar ist und die Einzelbilder in einem digitalen Bildformat bereitstellbar sind, **dadurch gekennzeichnet, dass** die Einzelbilder von der Datenverarbeitungsvorrichtung (20) in Echtzeit in dem Speicher (25) abspeicherbar sind, wobei der Speicher (25) als Ringspeicher (26) mit n Speicherplätzen (27.1, 27.2, ..., 27.24) ausgebildet ist, sodass das neuste Einzelbild in einem nächsten, freien Speicherplatz (27.1, 27.2, ..., 27.24) des Ringspeichers (26) abspeicherbar ist, solange in dem Ringspeicher (26) freie Speicherplätze (27.1, 27.2, ..., 27.24) vorhanden sind, und dass jeweils das älteste Einzelbild mit dem jeweils neusten Einzelbild überschreibbar ist, sobald alle n Speicherplätze (27.1, 27.2, ..., 27.24) des Ringspeichers (26) belegt sind.

11. Vorrichtung nach Anspruch 10, wobei die Bilderfassungsvorrichtung als Videokamera (15) ausgebildet ist.

12. Vorrichtung nach einem der Ansprüche 10-11, wobei der Ringspeicher (26) eine konfigurierbare Anzahl Speicherplätze (27.1, 27.2, ..., 27.24) umfasst und insbesondere als flüchtiger Speicher (25) ausgebildet ist.

13. Vorrichtung nach einem der Ansprüche 10-12 zur optischen Erfassung eines Auges (2) eines Patienten (3), wobei die Vorrichtung ein ophthalmologisches Untersuchungsgerät (4) umfasst und die Bilderfassungsvorrichtung (15) derart auf dem Untersuchungsgerät (4) montiert ist, dass eine optische Achse der Bilderfassungsvorrichtung (15) mit einer optischen Achse des Untersuchungsgeräts (4) zusammenfällt, das Untersuchungsgerät (4) eine Positioniervorrichtung (6) zur Positionierung des Untersuchungsgerätes (4) vor dem Auge (4) umfasst und die Bilderfassungsvorrichtung (15) mittels der Positioniervorrichtung (6) des Untersuchungsgeräts (4) vor dem Auge (2) positionierbar ist.

14. Vorrichtung nach einem der Ansprüche 10-13, wobei die Bilderfassungsvorrichtung (15) von der Datenverarbeitungsvorrichtung (20) steuerbar ist.
